# EUROPÄISCHE PATENTSCHRIFT

(11) **EP 1 257 250 B1**
(45) Veröffentlichungstag und Bekanntmachung des Hinweises auf die Patenterteilung: **25.05.2005**
(21) Anmeldenummer: 01927672.4
(22) Anmeldetag: 16.02.2001
(51) Int. Cl.: A61K 7/16

(54) **SYSTEM BESTEHEND AUS TREIBGASHALTIGEM ZAHNREINIGUNGSMITTEL UND SPENDER**
SYSTEM CONSISTING OF A DENTAL CLEANING AGENT CONTAINING PROPELLANT GAS AND A DISPENSER
SYSTEME CONSTITUE D'UN AGENT DE NETTOYAGE DENTAIRE CONTENANT DU GAZ PROPULSEUR ET D'UN DISTRIBUTEUR

(30) Priorität: 25.02.2000 DE 10008839
(43) Veröffentlichungstag der Anmeldung: 20.11.2002
(73) Patentinhaber: Henkel Kommanditgesellschaft auf Aktien, 40589 Düsseldorf (DE)
(72) Erfinder: LEINEN, Hans-Theo, 40229 Düsseldorf (DE); WÜLKNITZ, Peter, 42799 Leichlingen (DE); POSCHEN, Guido, 79599 Wittlingen (DE); BRESSLER, Christian, 60385 Frankfurt (DE)
(86) Internationale Anmeldenummer: PCT/EP2001/001724
(87) Internationale Veröffentlichungsnummer: WO 2001/062212

(56) Entgegenhaltungen:
- WO-A-82/03975
- DE-A- 2 526 808
- DE-A- 19 730 651
- DE-A- 19 834 355
- GB-A- 1 317 771
- GB-A- 1 465 495
- US-A- 2 968 628
- US-A- 2 995 521
- US-A- 5 292 500
- PATENT ABSTRACTS OF JAPAN vol. 006, no. 078 (C-102), 15. Mai 1982 (1982-05-15) & JP 57 014520 A (LION CORP), 25. Januar 1982 (1982-01-25) in der Anmeldung erwähnt

## Beschreibung

Die Erfindung betrifft ein System bestehend aus einem niedrig tensidhaltigen, treibmittelhaltigen Zahnreinigungsmittel und einem das treibmittelhaltige Zahnreinigungsmittel enthaltenden Spender mit Mitteln zur Bevorratung und Abgabe des Zahnreinigungsmittels, welche Mittel ein manuell betätigbares Abgabeventil umfassen.

Bei manueller Betätigung des Abgabeventiles wird das Zahnreinigungsmittel als Pastenoder Gelstrang abgegeben und quillt danach durch Freisetzung des Treibmittels langsam auf und entwickelt einen feinblasigen Schaum.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Cremeund transluzenter oder transparenter Gelform angeboten. In den letzten Jahren haben auch Liquid- oder Flüssigzahncremes und Mundwässer zunehmend an Bedeutung gewonnen. Ein Nachteil der Produkte in Pasten-, Creme- oder Gelform ist deren vergleichsweise hohe Viskosität und dadurch bedingte schlechte Verteilbarkeit und langsame Freisetzung der Wirk- und Aromastoffe.

Eine verbesserte Verteilbarkeit erreicht man durch aufgeschäumte Zahncremes, die üblicherweise aus Aerosolspendern abgegeben werden. Derartige Formulierungen sind aus der WO 99/22704, WO 82/03975, EP 0 208 009, AU 8542824, DE 2 217 953, DE 36 23 934, JP 5714520 A und der JP 5714521 A bekannt. Als Treibgase werden FCKW's, Kohlendioxid, Sauerstoff und niedrigsiedende Kohlenwasserstoffe eingesetzt. Der Einsatz von Fluor-Chlor-Kohlenwasserstoffen (FCKWs) ist wegen ihrer ökologischen Bedenklichkeit in vielen Ländern jedoch nicht mehr erlaubt. Generell handelt es sich bei Zahnpastaschäumen um Emulsionen oder Dispersionen, die aus den Spendern in bereits aufgeschäumter Form abgegeben werden. Dies hat den Nachteil, daß die Wirkstoffinenge oft viel zu niedrig ist. Derartige Zahnpastaschäume sind meist nur über kurze Zeit stabil. Auch hat dies den Nachteil, daß die bereits aufgeschäumte Masse nach Beendigung des Abgabevorganges teilweise in der Abgabeöffnung des Spenders austrocknet und diese verschmutzt. Darüber hinaus sind reine Kohlenwasserstofftreibgase mit den üblichen Zahnpastaformulierungen meist nicht kompatibel, da sie in diesen unlöslich sind. so daß es zu einer Phasentrennung kommt.

Ferner sind gelartige tensid- und treibmittelhaltige Formulierungen bekannt, die erst zeitlich verzögert nach ihrer Abgabe aus einem Spender aufschäumen. Diese Formulierungen sind durch einen relativ hohen Tensidgehalt und eine wasserähnliche Viskosität gekennzeichnet. Hohe Viskositäten werden vermieden, da diese in Abfüllanlagen zu niedrigen Abfüllgeschwindigkeiten und Taktzeiten führen. Zahnreinigungsmittel dieser Art sind nicht bekannt.

Der Erfindung lag daher die Aufgabe zu Grunde, eine Lösung zu schaffen, die die Bevorratung des Zahnreinigungsmittels und dessen Abgabe als pasten- oder gelartiger Strang mit zeitverzögertem Aufschäumen zu einem mousseartigen Schaum am Applikationsort ermöglicht.
Weitere Aufgabe war es, ein Zahnreinigungs- und -pflegemittel mit verbesserter Verteilbarkeit sowie vorzugsweise effizienter Wirkstoff- und Aromafreisetzung bereitzustellen, das zunächst als formbeständiger, nicht oder nur gering aufgeschäumter, pasten- oder gelartiger Strang aus einem Spender abgegeben wird, und dann allmählich, insbesondere erst bei Applikation im Mund, einen mousseartigen, "prickelnden" Schaum mit völlig neuartiger Sensorik und intensivierter Aromaentfaltung ausbildet.

Eine weitere Aufgabe bestand darin, die Zusammensetzung so zu optimieren, daß Treibgas und Zahnpastagrundlage eine stabile Dispersion ausbilden, die sich nicht trennt oder das Abgabe- und Leitungssystem des Spenders blockiert.

Diese Aufgabe wird erfindungsgemäß gelöst durch ein System bestehend aus einem tensidhaltigen Zahnreinigungsmittel enthaltend
a) wenigstens ein Poliermittel
b) wenigstens ein Feuchthaltemittel
c) wenigstens ein Tensid und
d) ein Treibmittel, insbesondere ein Treibgas oder Treibgasgemisch,
und einem das treibmittelhaltige Zahnreinigungsmittel enthaltenden Spender mit Mitteln zur Bevorratung und Abgabe des Zahnreinigungsmittels, dessen Mittel zur Bevorratung und Abgabe des Zahnreinigungsmittels ausreichend druckstabil gegenüber dem Expansionsdruck des treibmittelhaltigen Zahnreinigungsmittels bzw. des Treibmittels ausgebildet sind, und dessen Mittel zur Abgabe des treibmittelhaltigen Zahnreinigungsmittels ein eine Abgabeöffnung aufweisendes, manuell betätigbares Abgabeventil umfassen, wobei Zahnreinigungsmittel und Spender derart zusammenwirken, daß bei manueller Betätigung des Abgabeventils das treibmittelhaltige Zahnreinigungsmittel in strangförmiger, wenig aufgeschäumter Form aus der Abgabeöffnung austritt und zeitlich verzögert am Applikationsort aufschäumt, wobei das Zahnreinigungsmittel eine Viscosität größer gleich 30000 mPas (Brookfield Rotationsviskosimeter RVF, Spindel 3-5 bei 4 rpm und 25°C) aufweist und das in dem Zahnreinigungsmittel enthaltene Treibgas oder Treibgasgemisch wenigstens eine Komponente enthält, die bei Normaldruck im Bereich von -15 bis 35° C siedet.

Mit diesem System gelingt es, Zahnreinigungsmittel als pasten- oder gelartigen Strang mit zeitverzögertem Aufschäumen zu einem mousseartigen Schaum am Applikationsort zu applizieren. Die Abgabeöffnung des Spenders muß im Querschnitt ausreichend groß sein, um einen Strang des Mittels formend abzugeben.

In Ausgestaltung sieht die Erfindung vor, daß die Bevorratungs- und Abgabemittel des Spenders einen das Zahnreinigungsmittel enthaltenden, flexiblen Behälter oder Beutel umfassen, auf den innerhalb des Spenders von außen die Kontraktionskraft eines gedehnten Gummibehälters oder die Expansionskraft eines weiteren, komprimierten Treibgases oder Treibgasgemisches oder die Kraft eines bei oder nach Betätigung des Abgabeventiles auf dieses zu bewegten Kolbens einwirken.

Von Vorteil ist es gemäß Ausgestaltung der Erfindung, wenn das Zahnreinigungsmittel niedrig tensidhaltig mit einem Gehalt von 0,3 - 5 Gew.-%, insbesondere 0,3 - 3 Gew.-% ausgebildet ist.

Weiterhin sieht die Erfindung vor, daß das Zahnreinigungsmittel vorzugsweise eine Viskosität größer als 45.000 mPas, aufweist.

Auch kann in Weiterbildung der Erfindung der Treibmittelgehalt des Zahnreinigungsmittels kleiner gleich 10 Gew.-%, vorzugsweise kleiner gleich 4 Gew.-%, sein.

Überraschend hat sich gezeigt, daß mit diesem System trotz relativ niedrigen Tensidgehaltes des Zahnreinigungsmittels in Verbindung mit dessen relativ hohen Viskosität ein zunächst strangförmig aus dem Spender austretender und zeitverzögert aufschäumender Schaum oder eine Mousse sowohl optisch als auch sensorisch entsteht.

Der Anteil des Wassers an der Zusammensetzung beträgt 1 - 60 Gew.-%, vorzugsweise 5 - 50 Gew.-% und insbesondere 30 - 50 Gew.-%. Das Zahnreinigungsmittel weist üblicherweise einen pH-Wert von 5,5 - 9, vorzugsweise 6,5 - 8,0 und insbesondere 7,0 - 7,3 auf.

Die Zusammensetzung des Zahnreinigungsmittels ist zur Reinigung und Pflege der Zähne und zur Prävention von Zahn- und Zahnfleischerkrankungen geeignet. Das Zahnreinigungsmittel weist erfindungsgemäß ohne Treibgas bei 25° C eine Viskosität von größer gleich 30000 mPas, vorzugsweise größer 45000 mPas auf (Brookfield Rotationsviskosimeter RVF, Spindel 3 - 5 bei 4rpm), welche einerseits ein Abfüllen der Zusammensetzung beispielsweise über die Ventilkopföffnung eines Spenders ermöglicht und andererseits die Formbeständigkeit nach Entnahme des Mittels aus dem Spender gewährleistet, also ein Zerfließen verhindert. Das Mittel kann direkt, z. B. mit einer Zahnbürste appliziert werden, eignet sich jedoch bei Verdünnung mit Wasser auch besonders gut als Mundspülung, da der feine Schaum sehr leicht in Wasser dispergierbar ist. Damit die Zusammensetzungen zusammen mit dem Treibmittel bzw. Treibgas unter Verwendung handelsüblicher Abfüllanlagen beispielsweise durch die Ventilkopföffnung des jeweiligen Spenders abgefüllt werden können, ist es vorteilhaft, wenn die Viskosität des Mittels ohne Treibgas bei 25° C nicht höher als 100000 mPas ist, wobei ein Bereich von 30000 - 70000 mPas bevorzugt ist (Brookfield Rotationsviskosimeter RVF, Spindel 3 - 5; 4rpm).

Ein weiterer Gegenstand der Erfindung ist Verwendung eines ein treibmittel- und tensidhaltiges Mittel aufnehmenden Spenders mit Mitteln zur Bevorratung und Abgabe des Mittels, wobei die Bevorratungs- und Abgabemittel ausreichend druckstabil gegenüber dem Expansionsdruck des treibmittelhaltigen Mittels bzw. des Treibmittels sind und die Abgabemittel ein eine Abgabeöffnung aufweisendes, manuell zu betätigendes Abgabeventil umfassen, zur Bevorratung und Abgabe eines tensidhaltigen Zahnreinigungsmittels enthaltend ein Treibmittel, insbesondere ein Treibgas oder Treibgasgemisch.

Schließlich ist weiterer Gegenstand der Erfindung ein Verfahren zur Zahnpflege und - reinigung, bei welchem durch manuelle Betätigung eines Ventils aus einem Spendersystem ein nicht oder nur wenig aufgeschäumter Zahnpastastrang auf einen zur mechanischen Reinigung von Zähnen geeigneten Gegenstand aufgebracht wird, wobei der Zahnpastastrang bei 20° C und Normaldruck eine Expansionsrate von nicht mehr als 100 Vol.-% in 5 Sekunden aufweist und dann in einer Zeitspanne von 10 - 100 Sekunden auf nicht mehr als das 6-fache seines Volumens zu einer Zahnpasta-Mousse expandiert, und die gashaltige Zahnpasta-Mousse zur Reinigung von Zahnoberflächen, Zahnzwischenräume und der Mundhöhle verwendet wird.

### 1. Treibgas

Durch Verwendung eines Treibgases/Treibgasgemisches, welches anteilig wenigstens eine Komponente enthält, die bei Normaldruck im Bereich von -15 bis 35° C, insbesondere im Bereich von 9 bis 35 ° C und ganz bevorzugt im Bereich von 25 bis 35 ° C, siedet, erreicht man einen sogenannten "post-foaming" Effekt, der u. a. für die neuartige Sensorik und intensive Aromaentfaltung verantwortlich ist. Die erfindungsgemäße Zusammensetzung wird aus dem Spender in Form eines nicht oder nur gering aufgeschäumten, gelartigen Stranges abgegeben und bildet zeitverzögert, bei Entweichen des Treibgas/Treibgasgemisches - vorzugsweise im Mundraum - ein feinblasiges Mousse aus. Dies gewährleistet eine höhere Wirkstoffmenge im Vergleich zu Präparaten, die den Spendern direkt als voluminöser Schaum entnommen werden, und eine im Vergleich zu den üblichen Zahnreinigungsmitteln in Creme-, Gel- oder Pastenform verbesserte Verteilbarkeit der Wirkstoffe und optimierte Wirkstoff- und Aromafreisetzung. Die Treibgase sollten physiologisch unbedenklich sein, da ein Teil des Treibgases vorzugsweise erst bei Applikation des Mittels im Mundinnenraum freigesetzt wird. Beispielsweise sind kohlenwasserstoffhaltige Treibgase hierfür besonders vorteilhaft. In einer bevorzugten Ausführungsform enthält das System als Treibgas n-Butan oder Isobutan. Besonders bevorzugt ist ein Anteil an Treibgas-Komponenten, die im Bereich von 15 - 30° C und insbesondere 25 - 30° C sieden. Auf diese Weise läßt sich in Kombination mit erfindungsgemäßen und nachstehend beschriebenen Spendern ein "post-foaming" Effekt erzeugen.

Das Treibgas enthält in einer weiteren bevorzugten Ausführungsform ein C₅-Alkan, insbesondere in einer Menge von wenigstens 10 Gew.-% bezogen auf die Gesamtmenge des Treibgases. Unter diesen - n-Pentan, 2-Methylbutan (= Isopentan) und Neopentan - ist das 2-Methylbutan mit einem Siedepunkt von 29° C bevorzugt geeignet. Vorzugsweise wird als Treibgas ein Gemisch eines C₅-Alkans und eines C₄-Alkans eingesetzt, wobei 1:3 - 3:1-Gemische (Gewichtsverhältnis) aus Isopentan/Isobutan erfindungsgemäß besonders gut geeignet sind, um die zeitverzögerte Schaumbildung des Zahnreinigungsmittels zu gewährleisten. Derartige Formulierungen sind direkt nach der Abgabe aus dem Spender noch gelartig und quellen dann auf der Zahnbürste allmählich zu einem Schaum auf, der sich insbesondere erst bei Körpertemperatur, also im Mundinnenraum, relativ schnell und voll entfaltet. Die zeitverzögerte Gasfreisetzung im Mundinnenraum bewirkt neben einer völlig neuen Sensorik vor allem eine verbesserte Aromaentfaltung mit einem intensivierten Frischegefühl.

Vorzugsweise ist das Treibgas oder Treibgasgemisch in den erfindungsgemäßen Zusammensetzungen in einer Menge von weniger gleich 10 Gew.-%, vorzugsweise kleiner gleich 4 Gew.-%, aber wenigstens 0,5 Gew.-% enthalten. Der Anteil der Komponente mit einem Siedepunkt von -15 bis 35° C beträgt, wenn vorhanden, vorzugsweise wenigstens 10 Gew.-% bezogen auf die Gesamtmenge der Treibgaszusammensetzung. Ebenso bevorzugt ist es, den Gehalt an Treibgas nicht höher als 10 Gew.-% bezogen auf die Gesamtzusammensetzung zu wählen, da das Mittel ansonsten einen zu voluminösen Schaum bildet. Besonders bevorzugt ist es, 3 - 4 Gew.-% des Treibgases/Treibgasgemisches einzusetzen.

### 2. Spender

Das Zahnreinigungsmittel wird in handelsüblichen Abfüllanlagen zusammen mit einem durch Druck verflüssigten Treibgas oder Treibgasgemisch als Treibmittel in handbetätigbare Spender abgefüllt, die ausreichend druckfest bzw. druckstabil sind, so daß das Treibgas in der Zusammensetzung verflüssigt vorliegt. Als Spender für die Zusammensetzung eignet sich beispielsweise ein Behälter mit Ventil aus einem starren Material, der mit einer Vorrichtung ausgestattet ist, die zum Ausstoßen der in ihr bevorrateten Zusammensetzung (also Zahnreinigungsmittel und Treibgas) die Kontraktionskraft eines gedehnten Gummischlauches und/oder eines gedehnten Produktbehälters einsetzt. Ein solcher Spender ist beispielsweise in EP 69699 beschrieben. Auch die von der 3D Dispenser-Distributions GmbH unter der Bezeichnung FlexPack® angebotenen Behälter benutzen die Rückstellkraft eines gedehnten Gummis zum Ausstoßen der Zusammensetzung. Bevorzugt geeignet sind Behälter, die einen gefalteten, im wesentlichen gasundurchlässigen flexiblen Innenbeutel aus einem chemisch inerten Kunststoff (z. B. PET) enthalten, der von einem elastischen, dickwandigen Gummischlauch umgeben ist. Derartige Spender sind beispielsweise in US 5,927,551, US 4,964,540 und EP 69738 beschrieben und werden von der Firma Exxel Container unter der Bezeichnung Atmos™ Dispensing System vertrieben. In Kombination mit derartigen Spender kann auch reines Butan als Treibgas bereits einen "post-foaming" Effekt erzeugen.

Unter den Zweikammer-Druckpackungen sind Aerosoldosen mit Innenbeutel ebenso als Spender geeignet. Diese enthalten im Innenbeutel das Füllgut (Zahnreinigungsmittel mit verflüssigtem Gas) und in einer äußeren Kammer ein weiteres Treibgas, das zum Ausstoßen der Zusammensetzung bei Betätigung des Ventils dient. Derartige Systeme können entweder durch eine Öffnung am Dosenboden oder - wie beim sogenannten "under cup system" - durch Anheben des Ventils befüllt werden.

Auch geeignet sind handbetätigbare Kolbenspender, vorzugsweise mit Innenbeutel, bei welchen bei oder nach Betätigung des Abgabeventiles sich der Kolben auf das Ventil zu bewegt und dabei eine zur Abgabe des Zahnreinigungsmittels ausreichende Kraft auf das Zahnreinigungsmittel ausübt oder überträgt. Solche Spender ohne Innenbeutel werden für übliche Zahnpasten vielfach verwendet.

### 3. Oberflächenaktive Substanzen

Essentiell für die Reinigungswirkung und Schaumbildung der erfindungsgemäßen Zahnreinigungsmittel sind geeignete oberflächenaktive Tenside oder Tensidgemische. Diese tragen auch zu einer partiellen Solubilisierung des Treibgases/Treibgasgemisches bei und stabilisieren somit die gesamte Dispersion. Der Zusatz von Tensiden dient somit zur Erzeugung eines Schaums beim Zähnebürsten und damit zur besseren Wirkstoffverteilung, zur Stabilisierung der Poliermittel- und Treibgasdispersion sowie zur Emulgierung oder Solubilisierung der Fette, Öle, Wachse und der Aromaöle. Eine ausführliche Auflistung geeigneter Tenside ist in U.S. 3,988,433 enthalten. Das Tensid oder Tensidgemisch wird in den erfindungsgemäßen Zusammensetzungen in einer Menge von 0,3 - 5 Gew.-%, vorzugsweise 0,3 - 3 Gew.-%, bezogen auf die Gesamtzusammensetzung eingesetzt. Vorzugsweise enthält die Zusammensetzung wenigstens ein Tensid aus der Gruppe der Aniontenside, der Amphotenside und der Niotenside oder einem beliebigen Gemisch dieser Tenside.

### Aniontenside

Geeignete Tenside mit guter Schaumwirkung sind anionische Tenside, die auch eine gewisse enzymhemmende Wirkung auf den bakteriellen Stoffwechsel des Zahnbelags haben. Hierzu gehören Alkali- oder Ammoniumsalze - insbesondere Natriumsalze von C₈-C₁₈-Alkancarbonsäuren, von Alkylpolyglycolethersulfaten mit 12 - 16 C-Atomen in der linearen Alkylgruppe und 2 - 6 Glycolethergruppen im Molekül, von linearen Alkan-(C₁₂-C₁₈)-sulfonaten, Sulfobernsteinsäuremonoalkyl-(C₁₂-C₁₈)-estern, sulfatierten Fettsäuremonoglyceriden, sulfatierten Fettsäurealkanolamiden, Sulfoessigsäurealkyl-(C₁₂-C₁₆)-estem, Acylsarcosinen, Acyltauriden und Acylisethionaten mit jeweils 8 - 18 C-Atomen in der Acylgruppe. Bevorzugt ist die Verwendung wenigstens eines Aniontensids. insbesondere eines Natriumalkylsulfats mit 12 - 18 C-Atomen in der Alkylgruppe. Ein derartiges Tensid ist Natriumlaurylsulfat, das beispielsweise unter der Bezeichnung Texapon® K1296 im Handel ist.

### Zwitterionische und ampholytische Tenside

Auch zwitterionische und ampholytische Tenside können, bevorzugt in Kombination mit anionischen Tensiden, eingesetzt werden. Zu den erfindungsgemäß einsetzbaren Amphotensiden gehört z. B. Alkylaminopropancarbonsäure. Die bekannteste und am weitesten verbreitete Gruppe der zwitterionischen Tenside ist die der Betain-Tenside, wie z. B. AIkyldimethylcarboxymethylbetain und Acylaminoalkyldimethylcarboxymethylbetain. Bevorzugt geeignet sind Kokosacylaminopropyldimethylammoniumglycinate, die unter der INCI-Bezeichnung Cocamidopropylbetain bekannt sind. Solche Produkte sind beispielsweise unter der Bezeichnung Tego-Betain® BL 215 und ZF 50 sowie Genagen® CAB im Handel.

### Niotenside

Besonders geeignet zur Unterstützung der Reinigungswirkung sind nichtionogene Tenside, unter denen die Anlagerungsprodukte von Ethylenoxid an Fettalkohole, an Fettsäuren, an Fettsäuremonoglyceride, an Sorbitan-Fettsäuremonoester oder an Methylglucosid-Fettsäuremonoester bevorzugt sind. Die angelagerte Menge an Ethylenoxid sollte dabei so hoch sein, daß die Tenside wasserlöslich sind, d. h. es sollte wenigstens 1 g des Produktes in 1l Wasser bei 20°C klar löslich sein. Erfindungsgemäß bevorzugt als nichtionogenes Tensid sind ethoxylierte Glycerylmonoalkylester mit einem Ethoxylierungsgrad von 20-60 und einer C₈-C₁₈-Alkylkette, wie beispielsweise PEG-30-Glycerylstearat, das z. B. unter der Bezeichnung Tagat® S im Handel ist, und auch als Lösungsvermittler fungiert.

Als nichtionogene Tenside eignen sich aber auch Alkyl-(oligo)-glycoside mit 8 - 16 C-Atomen in der Alkylgruppe und einem Oligomerisationsgrad des Glycosidrestes von 1 - 4 Alkyl-(oligo)-glycoside, ihre Herstellung und Verwendung als oberflächenaktive Stoffe sind z. B. aus US-A-3,839,318, DE-A-20 36 472, EP-A-77 167 oder WO-A-93/10132 bekannt. Bezüglich des Glycosidrestes gilt, daß sowohl Monoglycoside (x = 1), bei denen ein Monosaccharidrest glycosidisch an einen Fettalkohol mit 10 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt. Bevorzugt eignet sich als Alkyl-(oligo)-glycosid ein Alkyl-(oligo)-glucosid der Formel RO(C₆H₁₀O)ₓ-H, in der R eine Alkylgruppe mit 12 bis 14 C-Atomen ist und x einen Mittelwert von 1 bis 4 hat. Auch Aminoxide sind als Tenside einsetzbar.

Um eine intensive Reinigungswirkung in Kombination mit guter Schaumbildung und eine ausreichende Solubilisierung der Treibgase zu erreichen, wird in den erfindungsgemäßen Zusammensetzungen vorzugsweise ein Gemisch aus Anion- und Niotensid oder ein Gemisch aus Aniontensid, Niotensid und Betain verwendet, beispielsweise Natriumlaurylsulfat/PEG-30-Glycerylstearat/Cocamidopropylbetain.

### Lösungsvermittler

Zur partiellen Solubilisierung der Treibgase und Geschmacksstoffe und der ggf. enthaltenen Öl- und Wachskomponenten sowie zur weiteren Stabilisierung der gesamten Dispersion dienen neben Ethanol insbesondere nichtionogene Lösungsvermittler aus der Gruppe der oberflächenaktiven Substanzen. Hierzu zählen, wie bereits erwähnt, insbesondere ethoxylierte Verbindungen mit einem Ethoyxlierungsgrad von 20 - 60. Besonders geeignet für diesen Zweck sind ethoxylierte Fettsäureglyceride, ethoxylierte Fettsäuresorbitanpartialester oder Fettsäurepartialester von Glycerin- oder Sorbitan-Ethoxylaten. Lösungsvermittler aus der Gruppe der ethoxylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind ethoxylierte Fettsäuresorbitanpartialester, z. B. Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Ethoxylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

### Kationische Tenside

Ferner können auch einige kationische Tenside wie z. B. Alkyltrimethylammoniumchlorid, Alkyldimethylbenzylammoniumchlorid, Alkylpyridiniumchlorid, Alkyldimethylhydroxyethylammoniumchlorid, Acylimidazoliniummethosulfate und Acyloxyethyltrimethylammoniumchlorid als Tenside in den erfindungsgemäßen Mitteln eingesetzt werden.

### 4. Feuchthaltemittel

Feuchthaltemittel werden Zahncremes nicht nur zugesetzt, um sie vor Austrocknung zu schützen, sie dienen auch der Konsistenzgebung und der Kältestabilität und sind essentieller Bestandteil der erfindungsgemäßen Zusammensetzung. Als Feuchthaltemittel können toxikologisch unbedenkliche Polyole eingesetzt werden, wie z. B. Sorbitol, Xylitol, Glycerin, Mannitol, 1,2-Propylenglycol oder Gemische dieser Polyole. Auch Polyethylenglycole mit Molekulargewichten von 400 - 2000 können anteilig als Feuchthaltemittelkomponenten enthalten sein. In einer bevorzugten Ausführungsform enthält das erfindungsgemäße Mittel als Feuchthaltemittel wenigstens Sorbitol, Glycerin oder Xylitol bzw. ein beliebiges Gemisch dieser Substanzen. Das Feuchthaltemittel oder das Gemisch aus Feuchthaltemitteln ist in der Gesamtzusammensetzung in einer Menge von 15 - 85 Gew.-%, vorzugsweise 20 - 70 Gew.-% und insbesondere 30 - 50 Gew.-% enthalten. Vorzugsweise enthält die erfindungsgemäße Zusammensetzung ein Gemisch aus Feuchthaltemitteln, insbesondere mit einem Anteil an Sorbit/Polyethylenglycol im Gewichtsverhältnis 10 : (0,1-1).

### 5. Poliermittel

Die erfindungsgemäße Zusammensetzung enthält wenigstens ein Poliermittel, das zur mechanischen Entfernung des Zahnbelages dient, ohne den Zahnschmelz oder das Dentin zu schädigen. Als Poliermittel, Putzkörper oder Abrasivmittel werden in der Regel wassenmlösliche anorganische Stoffe eingesetzt, die den Zahnbelag mechanisch entfernen, ohne den Zahnschmelz oder das Dentin zu schädigen. Besonders vorteilhaft kann die Verwendung sehr feinteiliger Poliermittel mit einer mittleren Korngröße von 1 - 200 µm, vorzugsweise 1 - 50 µm und insbesondere 1 - 10 µm sein. Das Poliermittel wird in den erfindungsgemäßen Zusammensetzungen in einer Menge von 5 - 50 Gew.-%, vorzugsweise 8 - 30 Gew.-% und insbesondere 10 - 20 Gew.-% bezogen auf die Gesamtzusammensetzung eingesetzt. Häufig ist es besonders vorteilhaft, eine Kombinationen von Poliermitteln zu verwenden.

Erfindungsgemäß geeignet sind neben Kieselsäuren, wasserunlösliche Metaphosphate wie z. B. Natriummetaphosphat, Calciumphosphate wie z. B. Tricalciumphosphat, Calciumhydrogenphosphat, Calciumhydrogenphosphat-Dihydrat und Calciumpyrophosphat, Calciumcarbonat (Kreide), Magnesiumcarbonat, Magnesiumdihydrogenphosphat, Trimagnesiumphosphat, Aluminiumoxid, calciniertes Aluminiumoxid, Aluminiumhydroxid und Aluminiumoxid-Hydrate, Hydroxylapatit sowie verschiedene Silikate. Auch Natriumhydrogencarbonat ist als Poliermittel erfindungsgemäß einsetzbar, insbesondere in Mischung mit anderen Poliermitteln.

Vorzugsweise enthält die Zusammensetzung als Poliermittel wenigstens eine Verbindung aus der Gruppe der Kieselsäuren oder Silikate. Unter den Kieselsäure-Poliermitteln sind Fällungs- und Gelkieselsäuren bevorzugt geeignet, da sie bei der Herstellung in ihren Eigenschaften breit variiert werden können und besonders gut mit Fluorid-Wirkstoffen verträglich sind. Sie eignen sich auch besonders gut für die Herstellung von Gel- oder Liquid-Zahncremes.

Gelkieselsäuren werden durch Umsetzung von Natriumsilikatlösungen mit starken wäßrigen Mineralsäuren unter Ausbildung eines Hydrosols, Alterung zum Hydrogel, Waschen und anschließendem Trocknen erzeugt. Erfolgt die Trocknung unter schonenden Bedingungen auf Wassergehalte von 15 - 35 Gew.-%, so werden sogenannte Hydrogelkieselsäuren erhalten, wie sie beispielsweise aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sogenannten Xerogels. Solche Xerogelkieselsäuren sind z. B. in US 3,538,230 beschrieben.

Fällungskieselsäuren werden aus wässrigen Alkalisilikat-Lösungen durch Ausfällung mit Mineralsäuren hergestellt und zwar unter Bedingungen, bei denen eine Aggregation zum Sol und Gel nicht eintreten kann. Geeignete Verfahren zur Herstellung von Fällungskieselsäuren sind z. B. in DE-OS 25 22 486 und in DE-OS 31 14 493 beschrieben.

Erfindungsgemäß geeignete Kieselsäurepoliermittel sind beispielsweise unter der Bezeichnung Sident® 8, Zeodent® 113 und 623 , Sorbosil® AC39, Tixosil® 123 und 73 erhältlich.

Unter den Silikaten sind verschiedene Aluminiumsilikate und Zirkoniumsilikate als Poliermittel bekannt. Auch Natrium-Aluminiumsilikate können als Poliermittel geeignet sein, wie z. B. synthetische Zeolithe, insbesondere Zeolith A.

Ferner können auch teilchenförmige organische Polymere, z. B. Polymethacrylat oder Polyethylen und Polypropylen einer mittleren Teilchengröße von ca. 5 - 15 µm und einem durchschnittlichen Molekulargewicht von 3000 g/mol als Putzkörper eingesetzt werden.

### 6. Bindemittel/Verdickungsmittel

Das erfindungsgemäße Mittel enthält in einer bevorzugten Ausführungsform zusätzlich wenigstens ein Binde- oder Verdickungsmittel. Binde- oder Verdickungsmittel wirken u. a. konsistenzregulierend und tragen dazu bei, eine Separation der flüssigen und festen Bestandteile zu verhindern. Sie sind in den erfindungsgemäßen Zusammensetzungen in einer Gesamtmenge von 0,1 - 5 Gew.-%, vorzugsweise 0,1 - 3 Gew.-% und insbesondere 0,5 - 2 Gew.-% enthalten. Verwendet werden beispielsweise natürliche und/oder synthetische wasserlösliche Polymere wie Alginate, Carragene, Agar-Agar, Guar-Gum, Gummi arabicum, Succinoglycan-Gum, Guarmehl, Johannisbrotkernmehl, Tragant, Karaya-Gummi, Xanthan, Pektine, Cellulose und deren ionogene und nicht-ionogene Derivate wie z. B. Carboxymethylcellulose, Hydroxyethylcellulose oder Methylhydroxypropylcellulose, hydrophob modifizierte Cellulosen, Stärke- und Stärkeether. Auch wasserlösliche Carboxyvinylpolymere (z. B. Carbopol®-Typen), Polyvinylalkohol, Polyvinylpyrrolidon und höhermolekulare Polyethylenglycole (insbesondere solche mit Molekulargewichten von 10² - 10⁶ D) eignen sich als Binde- und Verdickungsmittel. Ebenso können Schichtsilikate und feinteilige Kieselsäuren (Aerogelkieselsäuren und pyrogene Kieselsäuren) diese Funktion erfüllen. Bevorzugt geeignet als Bindemittel sind wasserunlösliche, nicht-derivatisierte pulverförmige Cellulosen, die beispielsweise von J. Rettenmaier & Söhne unter der Bezeichnung Arbocel® und Vitacel® angeboten werden. Arbocel® CGP 5000, eine hochviskose Paste aus Pulvercellulose mit thixotropen Eigenschaften ist ein besonders effektiver Verdicker, der auch bei niedriger Einsatzkonzentration stark konsistenzgebende Eigenschaften besitzt, gegen ionische Bestandteile inert ist, und sich gut mit weiteren Verdickungsmitteln kombinieren läßt.

Durch die Kombination der Komponenten a) - c) gemäß Anspruch 1 (Poliermittel, Feuchthaltemittel, Tensid) mit einer wasserunlöslichen, nicht-derivatisierten Cellulose wird eine stabile Dispersion des Treibgas/Treibgasgemisches in den erfindungsgemäßen Zusammensetzungen ermöglicht. Auch bei längerer Lagerung sind diese Zusammensetzungen gegen eine Separation des Treibgases/Treibgasgemisches stabil. Eine Blockierung oder Verstopfung des Abgabe- und Leitungssystems des Spenders tritt nicht ein. Die erfindungsgemäßen Zusammensetzungen bilden durch den Zusatz der Cellulose besonders stabile Schäume, die nicht zerfließen.

Als Konsistenzregulator ist wasserunlösliche, nicht-derivatisierte Cellulose Bestandteil einer bevorzugten Zusammensetzung, der die Bildung eines besonders stabilen Schaums gewährleistet. In Kombination mit Tensiden und Lösungsvermittlern ermöglicht sie eine stabile Dispersion des Treibgases in der Zahnpasta-Grundmasse. In der erfindungsgemäßen Zusammensetzung trägt sie auch dazu bei, eine Separation der flüssigen und festen Bestandteile zu verhindern. Die Cellulose wird üblicherweise in einer Menge von 0,01 - 5,0 Gew.-% Aktivsubstanz, vorzugsweise 0,01- 2,0 Gew.-% bezogen auf die Gesamtzusammensetzung eingesetzt.

Im Sinne der Erfindung wird unter wasserunlöslich eine Löslichkeit von weniger als 1 Gew.-% in Wasser bei 20 °C verstanden, d. h. daß in 100g einer gesättigten wässrigen Lösung bei 20°C weniger als 1 Gew.-% der Cellulose gelöst ist. Im Sinne der Erfindung werden Cellulosen als nicht-derivatisiert bezeichnet, deren Hydroxyl-Wasserstoff-Atome nicht durch andere chemische Gruppen substituiert sind.

Cellulose ist eine ubiquitäre pflanzliche Gerüstsubstanz. Sie kann in nativer oder gereinigter Form eingesetzt werden. Gereinigte Cellulose ist aufgrund ihrer guten physiologischen Verträglichkeit für den Einsatz in der Oralhygiene bevorzugt geeignet. Das Molekulargewicht der Cellulose liegt üblicherweise unter 800000, vorzugsweise bei 10000 - 700000 und insbesondere bei 20000 - 500000.

Bevorzugt geeignet sind mikrokristalline oder pulverförmige Cellulosen, die beispielsweise von J. Rettenmaier & Söhne unter der Bezeichnung Arbocel® und Vitacel® angeboten werden. Cellulose mit einer durchschnittlichen Primärpartikelgröße von weniger als 1 µm, insbesondere Arbocel® CGP 5000, eine hochviskose Paste aus Pulvercellulose mit thixotropen Eigenschaften, ist besonders bevorzugt geeignet. Arbocel®-Cellulosen sind äußerst effektive Verdicker, die auch bei sehr niedriger Einsatzkonzentration die Viskosität der Zusammensetzungen beträchtlich erhöhen, gegen ionische Bestandteile inert sind und sich gut mit weiteren Verdickungsmitteln kombinieren lassen. Durch Kombination mit Cellulosen vom Arbocel®-Typ erhält man Formulierungen, die einerseits dünnflüssig genug sind, um eine Abfüllung über die Ventilkopföffnung des Spendersystems zu ermöglichen und andererseits nach Entnahme aus dem Spendersystem einen stabilen Schaum mit Volumen entwickeln, der ein völlig neuartiges Mundgefühl bewirkt. Der Schaum, der sich aus dem Gelstrang entwickelt, ist über mehrere Stunden hinweg stabil.

### 7. Öl/Fett- und Wachskomponenten

In einer weiteren bevorzugten Ausführungsform enthält die erfindungsgemäße Zusammensetzung zusätzlich Öl/Fett- und/oder Wachskomponenten. In Kombination mit den Tensiden ermöglichen diese eine partielle Solubilisierung des Treibgases oder Treibgasgemisches und verleihen der Zahnpasta-Grundmasse Glanz und Geschmeidigkeit. Durch die Kombination der Komponenten a) und b) von Anspruch 1 ( Poliermittel und Feuchthaltemittel) mit Wachs- und/oder Öl-Komponenten sowie Tensiden oder Emulgatoren wird eine stabile Dispersion des Treibgas/Treibgasgemisches in den erfindungsgemäßen Zusammensetzungen ermöglicht. Auch bei längerer Lagerung sind die erfindungsgemäßen Zusammensetzungen gegen eine Separation des Treibgases/Treibgasgemisches stabil. Eine Blockierung des Leitungssystems des Spenders tritt nicht ein. Es können natürliche, chemisch modifizierte und synthetische Wachse, Fette und Öle alleine oder in beliebiger Kombination eingesetzt werden. Die Öl/Fett- und Wachskomponenten werden in einer Menge von 0,1 bis 10 Gew.-%, bezogen auf die Gesamtzusammensetzung, vorzugsweise 0,5 bis 5 Gew.-% und insbesondere 0,8 - 3 Gew.-% eingesetzt.

### Öle/Fette

Im Sinne der Erfindung werden hierunter Mono-, Di- und Triglyceride mit flüssiger bis fester Konsistenz sowie Kohlenwasserstoffe und Silikonöle verstanden, die natürlichen oder synthetischen Ursprungs sein können. Erfindungsgemäß ist es bevorzugt, die Ölkomponenten aus der Gruppe der Triglyceride und/oder Paraffinöle zu wählen. Diese können pflanzlichen, tierischen oder synthetischen Ursprungs sein.

Mono-, Di- und Triglyceride sind die Mono-, Di- und Triester von Fettsäuren mit Glycerin, also Acylglycerine, wobei das Glycerin mit gleichen oder mit unterschiedlichen Fettsäuren oder Fettsäurederivaten (z. B. Lecithin) verestert sein kann. Bei den Fettsäuren handelt es sich vorzugsweise um C₆-C₃₀-Fettsäuren, die gesättigt oder ungesättigt sowie verzweigt oder unverzweigt sein können. Hierzu gehören u. a. die Produkte der Veresterung von Glycerin mit natürlich vorkommenden Fettsäuren wie beispielsweise Capronsäure, Önanthsäure, Caprylsäure, Pelargonsäure, Caprinsäure, Undecansäure, Laurinsäure. Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure, Melissinsäure, 2-Ethylhexansäure, Isotridecansäure, Isostearinsäure, Palmitoleinsäure, Ölsäure, Elaidinsäure, Petroselinsäure, Elaeostearinsäure, Erucasäure, Linolsäure, Linolensäure, Arachidonsäure, Clupanodonsäure, Docosahexaensäure und Gadoleinsäure sowie deren technische Mischungen, die z. B. bei der Druckspaltung von natürlichen Fetten und Ölen oder der Dimerisierung von ungesättigten Fettsäuren anfallen. Besonders geeignet kann die Verwendung natürlicher Fette und Öle wie z. B. Rindertalg, Erdnußöl, Rüböl, Baumwollsaatöl, Sojaöl, Sonnenblumenöl, Palmöl, Palmkernöl, Leinöl, Mandelöl, Rizinusöl, Maisöl, Olivenöl, Rapsöl, Sesamöl, Kakaobutter und Kokosfett und dergleichen sein. Auch die hydrierten oder gehärteten Öle, z. B. hydriertes Sojaöl, Rizinusöl und Erdnußöl können verwendet werden.

Erfindungsgemäß einsetzbar sind dünn- und dickflüssige Silikonöle oder natürliche und synthetische Kohlenwasserstoffe wie beispielsweise dünn- bis dickflüssige Paraffinöle, Isohexadecan, Isoeicosan oder Polydecene, die unter der Bezeichnung Emery® 3004, 3006, 3010, Ethylflo® oder Nexbase® 2004G erhältlich sind. Auch der Einsatz sogenannter inverser Fette (z. B. Ester vicinaler Tricarbonsäuren mit C₆-C₃₀-Fettalkoholen) oder von Glyceryltrialkyethern ist hier denkbar.

### Wachse

Unter Wachsen werden natürlich oder künstlich gewonnene Stoffe mit folgenden Eigenschaften verstanden: sie sind von fester bis brüchig harter Konsistenz, grob bis feinkristallin, durchscheinend bis opak, jedoch nicht glasartig, und schmelzen oberhalb von 35° C ohne Zersetzung. Sie sind schon wenig oberhalb des Schmelzpunktes niedrigviskos und nicht fadenziehend und zeigen eine stark temperaturabhängige Konsistenz und Löslichkeit. Die häufigsten Vertreter aus der Gruppe der Wachse sind chemisch gesehen Ester aus Fettsäuren und höheren Fettalkoholen, die tierischen und pflanzlichen Ursprungs sind. Erfindungsgemäß verwendbar sind natürliche pflanzliche Wachse, wie z. B. Jojobaöl, Candelillawachs, Camaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Es kann auch vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie Paraffinwachse (z. B. Vaseline) und Mikrowachse. Als Wachskomponente sind auch chemisch modifizierte Wachse, wie z. B. Montanesterwachse, Sasolwachse und hydrierte Jojobawachse einsetzbar. Zu den synthetischen Wachsen, die erfindungsgemäß einsetzbar sind, zählen beispielsweise auch wachsartige Polyalkylenwachse und Silikonwachse.

Erfindungsgemäß einsetzbar sind auch synthetische Wachse, insbesondere die Ester aus C₆-C₃₀-Fettsäuren mit C₆-C₃₀-Fettalkoholen, die natürlichen oder synthetischen Ursprungs sein können. Sowohl die Fettsäure-Komponente als auch die Fettalkohol-Komponente kann geradkettig oder verzweigt und gesättigt oder einfach oder mehrfach ungesättigt sein.

Vorzugsweise wird die Wachskomponente gewählt aus der Gruppe der pflanzlichen oder der tierischen und/oder der Paraffinwachse bzw. einem beliebigen Gemisch dieser Wachse. Besonders bevorzugt ist die Verwendung von Bienenwachs oder Jojobaöl.

### 8. Zusätzliche Wirkstoffe

Eine weitere bevorzugte Ausführungsform des Zahnreinigungsmittels ist dadurch gekennzeichnet, daß es als zusätzliche Wirkstoffe Antikarieswirkstoffe, antimikrobielle Wirkstoffe, Zahnstein-Inhibitoren, Remineralisierungswirkstoffe, Geschmacksstoffe oder eine beliebige Kombination dieser Stoffe enthält.

### Antikaries-Wirkstoffe

Zur Bekämpfung von und Vorbeugung gegen Karies eignen sich vor allem Fluorverbindungen, bevorzugt aus der Gruppe der Fluoride oder Monofluorophosphate in einer Menge von 0,1 - 0,5 Gew.-% Fluor. Geeignete Fluorverbindungen sind z. B. Natriumfluorid, Kaliumfluorid, Zinnfluorid, Dinatriummonofluorophosphat (Na₂PO₃F), Dikaliummonofluorophosphat oder das Fluorid einer organischen Aminoverbindung.

### Antimikrobielle Wirkstoffe

Als antimikrobielle Komponente eignen sich z. B. Phenole, Resorcine, Bisphenole, Salicylanilide und -amide sowie deren halogenierte Derivate, halogenierte Carbanilide und p-Hydroxybenzoesäureester. Unter den antimikmbiellen Komponenten sind diejenigen besonders geeignet, die das Wachstum von Plaque-Bakterien hemmen. Beispielsweise sind halogenierte Diphenylether, wie 2,4-Dichlor-2'-hydroxydiphenylether, 4,4'-Dichlor-2'-hydroxydiphenylether, 2,4,4'-Tribrom-2'-hydroxydiphenylether, 2,4,4'-Trichlor-2'hydroxydiphenylether (Triclosan) als antimikrobielle Wirkstoffe geeignet. Neben Bromchlorophen, Bisbiguaniden wie Chlorhexidin und Alexidin, Phenylsalicylsäureestern und 5-Amino-1,3-bis(2-ethylhexyl)-hexahydro-5-methylpyrimidin (Hexetidin) wirken auch Zink- und Kupferionen antimikrobiell, wobei synergistische Effekte insbesondere in Kombination mit Hexetidin und Triclosan auftreten. Auch quartäre Ammoniumverbindungen, wie z. B. Cetylpyridiniumchlorid, Benzalkoniumchlorid, Domiphenbromid und Dequaliniumchlorid sind einsetzbar. Als antimikrobiell wirksam haben sich auch Octapinol, Octenidine und Sanguinarin erwiesen. Die antimikrobiellen Wirkstoffe werden bevorzugt in Mengen von 0,01 - 1 Gew.-% in den erfindungsgemäßen Mitteln eingesetzt. Besonders bevorzugt wird Irgacare® MP in einer Menge von 0,01 - 0,3 Gew.-% verwendet.

### Zahnsteininhibitoren

Bei Zahnstein handelt es sich um Mineralablagerungen, die dem natürlichen Zahnschmelz sehr ähnlich sind. Um eine Zahnsteinbildung zu inhibieren, werden den erfindungsgemäßen Zahnreinigungsmitteln Stoffe zugesetzt, die gezielt in die Kristallkeimbildung eingreifen und bereits vorhandene Keime am Weiterwachsen hindern. Hierbei handelt es sich beispielsweise um kondensierte Phosphate, die bevorzugt gewählt werden aus der Gruppe der Tripolyphosphate, der Pyrophophate, der Trimetaphosphate oder deren Gemischen. Sie werden in Form ihrer Alkali- oder Ammoniumsalze, bevorzugt in Form ihrer Natrium- oder Kaliumsalze eingesetzt. Wäßrige Lösungen dieser Phosphate reagieren typischerweise alkalisch, so daß der pH-Wert der erfindungsgemäßen Zahnpflegemittel ggf. durch Zusatz von Säure auf Werte von 7,5 - 9 eingestellt wird. Als Säuren können dabei z. B. Zitronensäure, Phosphorsäure oder saure Salze, z. B. NaH₂PO₄ verwendet werden. Der gewünschte pH-Wert des Zahnpflegemittels kann aber auch durch Zusatz saurer Salze der kondensierten Phosphate, also z. B. K₂H₂P₂O₇, eingestellt werden.

Auch Gemische verschiedener kondensierter Phosphate und/oder hydratisierte Salze der kondensierten Phosphate sind erfindungsgemäß einsetzbar. Zahnsteininhibitoren werden üblicherweise in Mengen von 0,1 - 5 Gew.-%, bevorzugt 0,1 - 3 Gew.-% und insbesondere 0,1 - 2 Gew.-% in den erfindungsgemäßen Mitteln eingesetzt.

Weitere geeignete Zahnsteininhibitoren sind Organophosphonate wie 1-Azacycloheptan-2,2-diphosphonat (Na-Salz), 1-Hydroxyethan-1,1-diphosphonat (Na-Salz) und Zinkcitrat.

### Remineralsierungswirkstoffe

Die erfindungsgemäßen Mittel enthalten vorzugsweise auch Stoffe, die eine Remineralisienmg des Zahnschmelzes fördern und Dentalläsionen zu schließen vermögen. Diese sind üblicherweise in einer Gesamtmenge von 0,1 - 10 Gew.-%, vorzugsweise 0,1 - 5 Gew.-% und insbesondere 0,1 - 3 Gew.-% enthalten. Hierzu gehören z. B. Fluoride, Phosphatsalze des Calciums wie z. B. Calciumglycerinphosphate, Calciumhydrogenphopsphat, Hydroxylapatit, Fluorapatit, F-dotierter Hydroxylapatit, Dicalciumphosphatdihydrat sowie Calciumfluorid. Aber auch Magnesiumsalze wie z. B. Magnesiumsulfat, Magnesiumfluorid oder Magnesiummonofluorophosphat wirken remineralisierend.

### Geschmacksstoffe

Vorzugsweise enthalten die erfindungsgemäßen Mittel Geschmacksstoffe, zu denen z. B. Süßungsmittel und/oder Aromaöle gehören. Als Süßungsmittel eignen sich beispielsweise Saccharinate (insbesondere Natriumsaccharinat), Cyclamate (insbesondere Natriumcyclamat) sowie Sucrose, Lactose, Maltose oder Fructose. Als Aromaöle kommen alle für Mund- und Zahnpflegemittel gebräuchlichen natürlichen und synthetischen Aromen in Frage. Natürliche Aromen können sowohl in Form der aus den Drogen isolierten ätherischen Öle (Mischung) als auch in Form der hieraus isolierten Einzelkomponenten verwendet werden. Bevorzugt sollte wenigstens ein Aromaöl aus der Gruppe Pfefferminzöl, Krausenminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine/mehrere daraus isolierte bzw. synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Eine besonders bevorzugte Ausführungsform des Zahnreinigungsmittel enthält a) 0,3 - 3 Gew.-% eines Tensids oder Tensidgemisches; b) 20 - 85 Gew.-% eines Feuchthaltemittels; c) 5 - 30 Gew.-% eines Poliermittel; d) 0,1 - 5 Gew.-% eines Binde- oder Verdikkungsmittels, e) 0,05 - 0,5 Gew.-% eines Fluorids; f) 5 - 50 Gew.-% Wasser g) 1 - 6 Gew.-% eines Treibgases oder Treibgasgemisches enthaltend wenigstens eine Komponente, die im Bereich von -15 bis 35 °C siedet, und ist in einem Spendersystem mit manuell betätigbarem Ventil abgefüllt. Die Kombination dieser Komponenten ermöglicht neben einem guten "post-foaming" Effekt eine sehr leichte Verteilbarkeit der Masse, eine gute Reinigungsleistung und bei Zugabe von Geschmacksstoffen eine besonders intensive Aromafreisetzung.

### Weitere übliche Inhaltsstoffe

Das erfindungsgemäße Mittel kann vorzugsweise eine Reihe weiterer Komponenten enthalten. Hierzu gehören u. a.:
- Vitamine, z. B. Retinol, Biotin, Tocopherol, Ascorbinsäure und deren Derivate (z. B. Ester, Salze);
- Pigmente, z. B. Titandioxid oder Zinkoxid;
- Bleichmittel;
- Farbstoffe;
- pH-Stellmittel und Puffersubstanzen, z. B. Natriumcitrat, Natriumbicarbonat oder Kalium- und Natriumphosphate,
- Natriumbenzoat;
- wundheilende und entzündungshemmende Stoffe wie z. B. Allantoin, Harnstoff, Panthenol, Azulen oder Kamillenextrakt, Acetylsalicylsäure-Derivate, Rhodanid; Wasserstoffperoxid
- Zink- und Mangansulfat

### Beispiele

Die Herstellung des Zahnpflegereinigungsmittels erfolgte unter Entgasung, d. h. im mäßigen Vakuum (≤ 50 mbar), bei Raumtemperatur.

### Allgemeine Vorschrift

Die Feuchthaltemittel, Kieselsäuren und ggf. weitere Poliermittel wurden zusammen mit entsalztem Wasser im Vakuum unter Rühren homogenisiert. Anschließend wurden die löslichen Salze und Farbstoffe zugegeben. Cellulose wurde - falls verwendet - in etwas Wasser suspendiert und in die Masse eingearbeitet. Organische Bindemittel (z. B. Xanthan gum etc.) wurden in PEG dispergiert und anschließend unter Rühren in die Masse eingearbeitet. Nach Quellung der Masse wurden die Ölkomponenten und Tenside. die vorher miteinander bei 60 - 85 ° C emulgiert worden waren, zugegeben, dann die Aromastoffe, und solange weitergerührt, bis eine homogene Masse mit glatter Struktur erhalten wurde.

Die Pastenmasse (90 - 99 Gew.-%) wurde zusammen mit 1 - 10 Gew.-% des Treibgases/Treibgasgemisches in das Atmos™ Dispensing System (70 oder 140 mL) der Exxel Container Inc. oder in eine Zweikammer-Aerosol-Dose unter den für solche Systeme üblichen Bedingungen abgefüllt (Abfülldruck: ca. 40 bar).

Die Mengenangaben in nachfolgenden Beispielen beziehen sich, soweit nicht anders angegeben, auf Gew.-% Aktivsubstanz der Gesamtzusammensetzung ohne Treibgas.

### Beispiel 1 (Zusammensetzung ohne Treibgas)

| | |
|---|---|
| 12 Gew.-% | Silica (z. B. Sident® 8) |
| 60 Gew.-% | Sorbitol (z. B. Neosorb® 70170 B; 70 Gew.-% in Wasser) |
| 1,2 Gew.-% | Natriumlaurylsulfat (z. B. Texapon® K 1296) |
| 1,0 Gew.-% | Cocamidopropylbetain (z. B. Tego-Betain® BL 215) |
| 0,5 Gew.-% | PEG-30-Glycerylstearat (z. B. Tagat® S) |
| 3 Gew.-% | Bienenwachs (z. B. Bienenwachs Typ 8100 DAB) |
| 1 Gew.-% | Polyethylenglykol MG = 1550 (z. B. Polywachs® 1550) |
| 0,55 Gew.-% | Carboxymethylcellulose (z. B. Cekol® 2000 H) |
| 0,2 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew.-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

96 Gew.-% Zahnpastamasse wurden mit 4 Gew.-% eines 3:1-Gemisches (Gewichtsanteile) aus Isopentan und Isobutan in eine Zweikammer-Aerosoldose abgefüllt.

### Beispiel 2 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Paraffinöl, dünnflüssig (z. B. Paraffinum perliquidum P 130) |
| 1 Gew.-% | Polyethylenglykol MG = 1550 (z. B. Polywachs® 1550) |
| 0,55 Gew.-% | Xanthan gum (z. B. Keltrol ® F) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

96 Gew.-% Zahnpastamasse wurden mit 4 Gew.-% n-Butan in eine Zweikammer-Aerosoldose abgefüllt.

### Beispiel 3 (Zusammensetzung ohne Treibgas)

| | |
|---|---|
| 12 Gew.-% | Silica (z. B. Sident® 8) |
| 46 Gew.-% | Sorbitol (z. B. Neosorb® 70/70 B; 70 Gew.-% in Wasser) |
| 10 Gew.-% | Xylitol |
| 1,2 Gew.-% | Natriumlaurylsulfat (z. B. Texapon® K 1296) |
| 1,0 Gew.-% | Cocamidopropylbetain (z. B. Tego-Betain® BL 215) |
| 0,5 Gew.-% | PEG-30-Glycerylstearat (z. B. Tagat® S) |
| 3 Gew.-% | Bienenwachs (z. B. Bienenwachs Typ 8100 DAB) |
| 1 Gew.-% | Polyethylenglykol MG = 1550 (z. B. Polywachs® 1550) |
| 0,55 Gew.-% | Carboxymethylcellulose (z. B. Cekol® 2000 H) |
| 0,2 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriurnbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

96 Gew.-% Zahnpastamasse wurden mit 4 Gew.-% eines 3:1-Gemisches (Gewichtsanteile) aus Isopentan und Isobutan in das Atmos™ Dispensing System von Exxel abgefüllt.

### Beispiel 4 (Zusammensetzung ohne Treibgas)

| | |
|---|---|
| 12 Gew.-% | Silica (z. B. Sident® 8) |
| 16 Gew.-% | Sorbitol (z. B. Neosorb® 70/70 B; 70 Gew.-% in Wasser) |
| 10 Gew.-% | Xylitol |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Paraffinöl (z. B. Paraffinum perliquidum P 130) |
| 1 Gew.-% | Polyethylenglykol MG = 1550 (z. B. Polywachs® 1550) |
| 0,55 Gew.-% | Xanthan gum (z. B. Keltrol® F) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

97 Gew.-% Zahnpastamasse wurden mit 3 Gew.-% eines 3:1-Gemisches (Gewichtsanteile) aus Isopentan und Isobutan in das Atmos™ Dispensing System von Exxel abgefüllt.

### Beispiel 5 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 1 Gew.-% | Polyethylenglykol MG = 1550 (z. B. Polywachs® 1550) |
| 0,55 Gew.-% | Xanthan gum (z. B. Keltrol ® F) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

98 Gew.-% Zahnpastamasse wurden mit 2 Gew.-% n-Butan in eine Zweikammer-Aerosoldose abgefüllt.

### Beispiel 6 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Paraffinöl, dickflüssig (z. B. BP-Energol® WM-6) |
| 1 Gew.-% | Polyethylenglykol MG = 1550 (z. B. Polywachs® 1550) |
| 0,55 Gew.-% | Carboxymethylcellulose (z. B. Cekol® 2000H) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

97 Gew.-% Zahnpastamasse wurden mit 3 Gew.-% eines 2:2-Gemisches (Gewichtsanteile) aus Isopentan und Isobutan in das Atmos™ Dispensing System von Exxel abgefüllt.

### Beispiel 7 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Paraffinöl, dickflüssig (z. B. BP-Energol® WM-6) |
| 0,25 Gew.-% | Glycerinmonostearat (z. B. Cutina® GMS-V) |
| 1 Gew.-% | Polyethylenglykol MG = 1550 (z. B. Polywachs® 1550) |
| 0,45 Gew.-% | Xanthan gum (z. B. Keltrol® F) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

96 Gew.-% Zahnpastamasse wurden mit 4 Gew.-% eines 3:1-Gemisches (Gewichtsanteile) aus Isopentan und Isobutan in eine Zweikammer-Aerosoldose abgefüllt.

### Beispiel 8 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Paraffinöl, dickflüssig (z. B. BP-Energol® WM-6) |
| 0,35 Gew.-% | Glycerinmonostearat (z. B. Cutina® GMS-V) |
| 1 Gew.-% | Polyethylenglykol MG = 1550 (z. B. Polywachs® 1550) |
| 0,45 Gew.-% | Xanthan gum (z. B. Keltrol ® F) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

97 Gew.-% Zahnpastamasse wurden mit 3 Gew.-% eines 3:1-Gemisches (Gewichtsanteile) aus Isopentan und Isobutan in das Atmos™ Dispensing System von Exxel abgefüllt.

### Beispiel 9 (Zusammensetzung ohne Treibgas)

| | |
|---|---|
| 12 Gew.-% | Silica (z. B. Sident® 8) |
| 60 Gew.-% | Sorbitol (z. B. Neosorb® 70/70 B; 70 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 1,5 Gew.-% | Polyethylenglykol MG = 1550 (z. B. Polywachs® 1550) |
| 1 Gew.-% | Cellulose (z. B. Arbocel® CGP 5000; 5 Gew.-% in Wasser) |
| 0,55 Gew.-% | Xanthan gum (z. B. Keltrol® F) |
| 0,2 Gew.-% | Na₂HPO₄ |
| 0,32 Gew.-% | NaF |
| 0,2 Gew.-% | Natriumsaccharinat |
| 0,49 Gew,-% | Natriumbenzoat |
| 0,1 Gew.-% | Triclosan (z. B. Irgacare® MP) |
| 0,26 Gew.-% | Farbstofflösung (z. B. Kombination aus: Brillant Blue FCF, 1 Gew.-% in Wasser und Schwarz CI 27755, 0,1 Gew.-% in Wasser) |
| 1 Gew.-% | Aromaöl |
| ad 100 Gew.-% | Wasser VE |

97 Gew.-% Zahnpastamasse wurden mit 3 Gew.-% eines 3:1-Gemisches (Gewichtsanteile) aus Isopentan und Isobutan in das Atmos™ Dispensing System von Exxel abgefüllt.

### Beispiel 10 (Zusammensetzung ohne Treibgas)

| | |
|---|---|
| 12 Gew.-% | Silica (z. B. Sident® 8) |
| 0,5 Gew.-% | Schichtsilikat (z. B. Laponite® DF) |
| 60 Gew.-% | Sorbitol (z. B. Neosorb® 70/70 B; 70 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 1,5 Gew.-% | Polyethylenglykol MG = 1550 (z. B. Polywachs® 1550) |
| 0,55 Gew.-% | Xanthan gum (z. B. Keltrol® F) |
| 0,2 Gew.-% | Na₂HPO₄ |
| 0,32 Gew.-% | NaF |
| 0,2 Gew.-% | Natriumsaccharinat |
| 0,49 Gew,-% | Natriumbenzoat |
| 0,1 Gew.-% | Triclosan (z. B. Irgacare® MP) |
| 0,26 Gew.-% | Farbstofflösung (z. B. Kombination aus: Brillant Blue FCF, 1 Gew.-% in Wasser und Schwarz CI 27755, 0,1 Gew.-% in Wasser) |
| 1 Gew.-% | Aromaöl |
| ad 100 Gew.-% | Wasser VE |

96 Gew.-% Zahnpastamasse wurden mit 4 Gew.-% eines 3:1-Gemisches (Gewichtsanteile) aus Isopentan und Isobutan in eine Zweikammer-Aerosoldose abgefüllt.

### Beispiel 11 (Zusammensetzung ohne Treibgas)

| | |
|---|---|
| 12 Gew.-% | Silica (z. B. Sident® 8) |
| 60 Gew.-% | Sorbitol (z. B. Neosorb® 70/70 B; 70 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 1 Gew.-% | Cellulose (z. B. Arbocel® CGP 5000; 5 Gew.-% in Wasser) |
| 0,65 Gew.-% | Xanthan gum (z. B. Keltrol ® F) |
| 1,5 Gew.-% | Polyethylenglykol MG = 1550 (z. B. Polywachs® 1550) |
| 0,2 Gew.-% | Na₂HPO₄ |
| 0,32 Gew.-% | NaF |
| 0,2 Gew.-% | Natriumsaccharinat |
| 0,49 Gew,-% | Natriumbenzoat |
| 0, 1 Gew.-% | Triclosan (z. B. Irgacare® MP) |
| 0,26 Gew.-% | Farbstofflösung (z. B. Kombination aus: Brillant Blue FCF, 1 Gew.-% in Wasser und Schwarz CI 27755, 0,1 Gew.-% in Wasser) |
| 1 Gew.-% | Aromaöl |
| ad 100 Gew.-% | Wasser VE |

93 Gew.-% Zahnpastamasse wurden mit 7 Gew.-% eines 3:1-Gemisches (Gewichtsanteile) aus Isopentan und Isobutan in das Atmos™ Dispensing System von Exxel abgefüllt.

### Beispiel 12 (Zusammensetzung ohne Treibgas)

| | |
|---|---|
| 12 Gew.-% | Silica (z. B. Sident® 8) |
| 60 Gew.-% | Sorbitol (z. B. Neosorb® 70/70 B; 70 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1,5 Gew.-% | Polyethylenglykol MG = 1550 (z. B. Polywachs® 1550) |
| 0,75 Gew.-% | Cellulose (z. B. Arbocel® CGP 5000; 5 Gew.-% in Wasser) |
| 0,3 Gew.-% | Xanthan gum (z. B. Keltrol ® F) |
| 0,2 Gew.-% | Na₂HPO₄ |
| 0,32 Gew.-% | NaF |
| 0,2 Gew.-% | Natriumsaccharinat |
| 0,49 Gew,-% | Natriumbenzoat |
| 0,1 Gew.-% | Triclosan (z. B. Irgacare® MP) |
| 0,26 Gew.-% | Farbstofflösung (z. B. Kombination aus: Brillant Blue FCF, 1 Gew.-% in Wasser und Schwarz CI 27755, 0,1 Gew.-% in Wasser) |
| 1 Gew.-% | Aromaöl |
| ad 100 Gew.-% | Wasser VE |

97 Gew.-% Zahnpastamasse wurden mit 3 Gew.-% Butangas in das Atmos™ Dispensing System von Exxel abgefüllt.

### Beispiel 13 (Zusammensetzung ohne Treibgas)

| | |
|---|---|
| 12 Gew.-% | Silica (z. B. Sident® 8) |
| 60 Gew.-% | Sorbitol (z. B. Neosorb® 70/70 B; 70 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 1,5 Gew.-% | Polyethylenglykol MG = 1550 (z. B. Polywachs® 1550) |
| 1 Gew.-% | Cellulose (z. B. Arbocel® CGP 5000; 5 Gew.-% in Wasser) |
| 0,55 Gew.-% | Xanthan gum (z. B. Keltrol ® F) |
| 0,2 Gew.-% | Na₂HPO₄ |
| 0,32 Gew.-% | NaF |
| 0,2 Gew.-% | Natriumsaccharinat |
| 0,49 Gew,-% | Natriumbenzoat |
| 0,1 Gew.-% | Triclosan (z. B. Irgacare® MP) |
| 0,26 Gew.-% | Farbstofflösung (z. B. Kombination aus: Brillant Blue FCF, 1 Gew.-% in Wasser und Schwarz CI 27755, 0,1 Gew.-% in Wasser) |
| 1 Gew.-% | Aromaöl |
| ad 100 Gew.-% | Wasser VE |

96 Gew.-% Zahnpastamasse wurden mit 4 Gew.-% Butan in eine Zweikammer-Aerosoldose abgefüllt.

### Beispiel 14 (Zusammensetzung ohne Treibgas)

| | |
|---|---|
| 12 Gew.-% | Silica (z. B. Sident® 8) |
| 60 Gew.-% | Sorbitol (z. B. Neosorb® 70/70 B; 70 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 1 Gew.-% | Cellulose (z. B. Arbocel® CGP 5000; 5 Gew.-% in Wasser) |
| 0,65 Gew.-% | Xanthan gum (z. B. Cekol® 2000 H) |
| 1,5 Gew.-% | Polyethylenglykol MG = 1550 (z. B. Polywachs® 1550) |
| 0,2 Gew.-% | Na₂HPO₄ |
| 0,32 Gew.-% | NaF |
| 0,2 Gew.-% | Natriumsaccharinat |
| 0,49 Gew,-% | Natriumbenzoat |
| 0,1 Gew.-% | Triclosan (z. B. Irgacare® MP) |
| 0,26 Gew.-% | Farbstofflösung (z. B. Kombination aus: Brillant Blue FCF, 1 Gew.-% in Wasser und Schwarz CI 27755, 0,1 Gew.-% in Wasser) |
| 1 Gew.-% | Aromaöl |
| ad 100 Gew.-% | Wasser VE |

98 Gew.-% Zahnpastamasse wurden mit 2 Gew.-% eines 3:1-Gemisches (Gewichtsanteile) aus Isopentan und Isobutan in das Atmos™ Dispensing System von Exxel abgefüllt.

### Beispiel 15 (Zusammensetzung ohne Treibgas)

| | |
|---|---|
| 12 Gew.-% | Silica (z. B. Sident® 8) |
| 60 Gew.-% | Sorbitol (z. B. Neosorb® 70/70 B; 70 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 2 Gew.-% | Cellulose (z. B. Vitacel® L 600) |
| 0,7 Gew.-% | Xanthan gum (z. B. Cekol® 2000 H) |
| 0,03 Gew.-% | Polyvinylpyrrolidin (z. B. Luviskol®K 30) |
| 1,5 Gew.-% | Polyethylenglykol MG = 1550 (z. B. Polywachs® 1550) |
| 0,2 Gew.-% | Na₂HPO₄ |
| 0,32 Gew.-% | NaF |
| 0,2 Gew.-% | Natriumsaccharinat |
| 0,49 Gew,-% | Natriumbenzoat |
| 0,10 Gew.-% | Triclosan (z. B. Irgacare® MP) |
| 0,02 Gew.-% | Farbstoff (z. B. Sicumet Blau CI 74160) |
| 1 Gew.-% | Aromaöl |
| ad 100 Gew.-% | Wasser VE |

96 Gew.-% Zahnpastamasse wurden mit 4 Gew.-% eines 3:1-Gemisches (Gewichtsanteile) aus Isopentan und Isobutan in eine Zweikammer-Aerosoldose abgefüllt.

### Beispiel 16 (Zusammensetzung ohne Treibgas)

| | |
|---|---|
| 12 Gew.-% | Silica (z. B. Sident® 8) |
| 46 Gew.-% | Sorbitol (z. B. Neosorb® 70/70 B; 70 Gew.-% in Wasser) |
| 10 Gew.-% | Xylitol |
| 1,2 Gew.-% | Natriumlaurylsulfat (z. B. Texapon® K 1296) |
| 1,0 Gew.-% | Cocamidopropylbetain (z. B. Tego-Betain® BL 215) |
| 0,5 Gew.-% | PEG-30-Glycerylstearat (z. B. Tagat® S) |
| 3 Gew.-% | Bienenwachs (z. B. Bienenwachs Typ 8100 DAB) |
| 1 Gew.-% | Polyethylenglykol MG = 1550 (z. B. Polywachs® 1550) |
| 0,55 Gew.-% | Carboxymethylcellulose (z. B. Cekol® 2000 H) |
| 0,2 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

96 Gew.-% Zahnpastamasse wurden mit 4 Gew.-% Butan in eine Zweikammer-Aerosoldose abgefüllt.

### Beispiel 17 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 1 Gew.-% | Polyethylenglykol MG = 1550 (z. B. Polywachs® 1550) |
| 0,55 Gew.-% | Xanthan gum (z. B. Keltrol® F) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

98 Gew.-% Zahnpastamasse wurden mit 2 Gew.-% eines 3:1-Gemisches (Gewichtsanteile) aus Isopentan und Isobutan in das Atmos™ Dispensing System von Exxel abgefüllt.

### Beispiel 18 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Paraffinöl, dickflüssig (z. B. BP-Energol® WM-6) |
| 1 Gew.-% | Polyethylenglykol MG = 1550 (z. B. Polywachs® 1550) |
| 0,55 Gew.-% | Carboxymethylcellulose (z. B. Cekol ® 2000H) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

97 Gew.-% Zahnpastamasse wurden mit 3 Gew.-% eines 3:1-Gemisches (Gewichtsanteile) aus Isopentan und Isobutan in eine Zweikammer-Aerosoldose abgefüllt.

### Beispiel 19 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Paraffinöl, dickflüssig (z. B. BP-Energol® WM-6) |
| 0,25 Gew.-% | Glycerinmonostearat (z. B. Cutina® GMS-V) |
| 1 Gew.-% | Polyethylenglykol MG = 1550 (z. B. Polywachs® 1550) |
| 0,45 Gew.-% | Xanthan gum (z. B. Keltrol ® F) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

96 Gew.-% Zahnpastamasse wurden mit 4 Gew.-% eines 3:1-Gemisches (Gewichtsanteile) aus Isopentan und Isobutan in das Atmos™ Dispensing System von Exxel abgefüllt.

### Beispiel 20 (Zusammensetzung ohne Treibgas)

| | |
|---|---|
| 12 Gew.-% | Silica (z. B. Sident® 8) |
| 30 Gew.-% | Sorbitol (z. B. Neosorb® 70/70 B; 70 Gew.-% in Wasser) |
| 33 Gew.-% | Glycerin (86 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Paraffinöl, dickflüssig (z. B. BP-Energol® WM-6) |
| 0,35 Gew.-% | Glycerinmonostearat (z. B. Cutina® GMS-V) |
| 1 Gew.-% | Polyethylenglykol MG = 1550 (z. B. Polywachs® 1550) |
| 0,45 Gew.-% | Xanthan gum (z. B. Keltrol ® F) |
| 0,5 Gew.-% | Na₂HPO₄ |
| 0,32 Gew.-% | NaF |
| 0,21 Gew.-% | MgSO₄ · 7 H₂O |
| 0,09 Gew.-% | ZnSO₄ · 7 H₂O |
| 0,01 Gew.-% | MnSO₄ · 1 H₂O |
| 0,2 Gew.-% | Natriumsaccharinat |
| 0,49 Gew,-% | Natriumbenzoat |
| 2 Gew.-% | Ethanol |
| 0,2 Gew.-% | Triclosan (z. B. Irgacare® MP) |
| 0,1 Gew.-% | Farbstofflösung (z. B. Brillant Blue FCF, 1 Gew.-% in Wasser) |
| 1,1 Gew.-% | Aromaöl |
| ad 100 Gew.-% | Wasser VE |

96 Gew.-% Zahnpastamasse wurden mit 4 Gew.-% Butan in das eine Zweikammer-Aerosoldose abgefüllt.

### Beispiel 21 (Zusammensetzung ohne Treibgas)

| | |
|---|---|
| 12 Gew.-% | Silica (z. B. Sident® 8) |
| 0,5 Gew.-% | Schichtsilikat (z. B. Laponite® DF) |
| 60 Gew.-% | Sorbitol (z. B. Neosorb® 70/70 B; 70 Gew.-% in Wasser) |
| 1,5 Gew.-% | Natriumlaurylsulfat (z. B. Texapon® K 1296) |
| 0,6 Gew.-% | Cocamidopropylbetain (z. B. Tego-Betain® BL 215) |
| 0,5 Gew.-% | PEG-30-Glycerylstearat (z. B. Tagat® S) |
| 1 Gew.-% | Jojobaöl |
| 1,5 Gew.-% | Polyethylenglykol MG = 1550 (z. B. Polywachs® 1550) |
| 0,55 Gew.-% | Xanthan gum (z. B. Keltrol® F) |
| 0,2 Gew.-% | Na₂HPO₄ |
| 0,32 Gew.-% | NaF |
| 0,2 Gew.-% | Natriumsaccharinat |
| 0,49 Gew,-% | Natriumbenzoat |
| 0,1 Gew.-% | Triclosan (z. B. Irgacare® MP) |
| 0,26 Gew.-% | Farbstofflösung (z. B. Kombination aus: Brillant Blue FCF, 1 Gew.-% in Wasser und Schwarz CI 27755, 0,1 Gew.-% in Wasser) |
| 1 Gew.-% | Aromaöl |
| ad 100 Gew.-% | Wasser VE |

97 Gew.-% Zahnpastamasse wurden mit 3 Gew.-% eines 3:1-Gemisches (Gewichtsanteile) aus Isopentan und Isobutan in das Atmos™ Dispensing System von Exxel abgefüllt.

### Anhang

1) Arbocel® CGP 5000
   Cellulose-Paste (5 Gew.-% in Wasser)
   Hersteller: J. Rettenmaier & Söhne GmbH + CO
2) Bienenwachs Typ 8100 DAB
   INCI: Cera alba
   Hersteller: Kahl & Co
3) Brilliant Blue® FCF
   INCI: Acid blue 9/ CI 42090
   Hersteller: Fiorio Colori S.p.A. (Pharmorgana)
4) BP-Energol® WM-6
   Paraffinöl, dickflüssig
   Hersteller: BP
5) Cekol® 2000 H
   INCI: Cellulose (Na-Carboxymethylcellulose)
   Hersteller: Metsa (Nordmann Rassmann)
6) Cutina® GMS-V
   INCI: Glyceryl stearate
   Hersteller: Cognis Deutschland GmbH (Henkel)
7) Irgacare® MP
   INCI: Triclosan
   Hersteller: Ciba
8) Keltrol® F
   INCI: Xanthan Gum
   Hersteller: Kelco
9) Laponite® DS
   Natrium-Magnesium-Lithium-Schichtsilikat
   Hersteller: Laporte
10)Neosorb® 70/70 B
   INCI: Sorbitol (70 Gew.-% in Wasser)
   Hersteller: Roquette
11) Paraffinum perliquidum P 130
   Paraffinöl, Viskosität: 17 mm²/s nach DIN 51562/1
   Hersteller: Parafluid Mineralölgesellschaft
12) Polywachs® 1550
   Polyethylenglykol, MG = 1550
   Hersteller: RWE/DEA (Contensio, ehemals Hüls)
13) Sident® 8
   INCI: Hydrated silica
   Hersteller: Degussa-Hüls
14) Tagat® S
   INCI: PEG-30 Glyceryl Stearate
   Hersteller: Goldschmidt
15) Tego-Betain® BL 215 (30 Gew.-% in Wasser)
   INCI: Cocamidopropyl betaine
   Hersteller: Goldschmidt
16) Texapon® K 1296
   INCI: Sodium lauryl sulfate
   Hersteller: Cognis Deutschland GmbH (Henkel)
17) Luviskol® K 30
   INCI: PVP
   Hersteller: BASF
18) Vitacel® L 600
   Cellulose-Pulver
   Hersteller: J. Rettenmaier & Söhne GmbH + CO

## Patentansprüche

1. System bestehend aus einem tensidhaltigen Zahnreinigungsmittel enthaltend
a) wenigstens ein Poliermittel
b) wenigstens ein Feuchthaltemittel
c) wenigstens ein Tensid und
d) ein Treibmittel, insbesondere ein Treibgas oder Treibgasgemisch,
und einem das treibmittelhaltige Zahnreinigungsmittel enthaltenden Spender mit Mitteln zur Bevorratung und Abgabe des Zahnreinigungsmittels, dessen Mittel zur Bevorratung und Abgabe des Zahnreinigungsmittels ausreichend druckstabil gegenüber dem Expansionsdruck des treibmittelhaltigen Zahnreinigungsmittels bzw. des Treibmittels ausgebildet sind, und dessen Mittel zur Abgabe des treibmittelhaltigen Zahnreinigungsmittels ein eine Abgabeöffnung aufweisendes, manuell betätigbares Abgabeventil umfassen, wobei Zahnreinigungsmittel und Spender derart zusammenwirken, daß bei manueller Betätigung des Abgabeventils das treibmittelhaltige Zahnreinigungsmittel in strangförmiger, wenig aufgeschäumter Form aus der Abgabeöffnung austritt und zeitlich verzögert am Applikationsort aufschäumt, wobei das Zahnreinigtmgsmittel eine Viskosität größer gleich 30.000 mPas (Brookfield Rotationsviskosimeter RVF, Spindel 3 - 5 bei 4 rpm und 25°C) aufweist und das in dem Zahnreinigungsmittel enthaltene Treibgas oder Treibgasgemisch wenigstens eine Komponente enthält, die bei Normaldruck im Bereich von -15 bis 35° C siedet..

2. System nach Anspruch 1, **dadurch gekennzeichnet,**
**daß** die Bevorratungs- und Abgabemittel des Spenders einen das Zahnreinigungsmittel enthaltenden, flexiblen Behälter oder Beutel umfassen, auf den innerhalb des Spenders von außen die Kontraktionskraft eines gedehnten Gummibehälters oder die Expansionskraft eines weiteren, komprimierten Treibgases oder Treibgasgemisches oder die Kraft eines bei oder nach Betätigung des Abgabeventiles auf dieses zu bewegten Kolbens einwirken.

3. System nach Anspruch 1 bis 3, **dadurch gekennzeichnet,**
**daß** das Zahnreinigungsmittel niedrig tensidhaltig mit einem Gehalt von mindestens 0,3 Gew.-% und maximal 3 Gew.-% ausgebildet ist.

4. System nach einem der vorangehenden Ansprüche, **dadurch gekennzeichnet,**
**daß** das Zahnreinigungsmittel eine Viskosität größer als 45.000 mPas (Brookfield Rotationsviskosimeter RVF, Spindel 3 - 5 bei 4 rpm und 25°C), aufweist.

5. System nach einem der vorangehenden Ansprüche, **dadurch gekennzeichnet,**
**daß** der Treibmittelgehalt des Zahnreinigungsmittels kleiner gleich 10 Gew.-%, vorzugsweise kleiner gleich 4 Gew.-%, ist.

6. System nach einem der vorangehenden Ansprüche, **dadurch gekennzeichnet,**
**daß** das Zahnreinigungsmittel als Treibgas n-Butan oder Isobutan enthält.

7. System nach einem der vorangehenden Ansprüche, **dadurch gekennzeichnet,**
**daß** das Zahnreinigungsmittel als Treibgas-Komponente ein C₅-Alkan in einer Menge von wenigstens 10 Gew.-% bezogen auf die Gesamtmenge der Treibgaszusammensetzung enthält.

8. System nach einem der vorangehenden Ansprüche, **dadurch gekennzeichnet,**
**daß** das in dem Zahnreinigungsmittel enthaltene Treibgasgemisch aus einem Gemisch eines C₄-Alkans und eines C₅-Alkans besteht.

9. System nach einem der vorangehenden Ansprüche, **dadurch gekennzeichnet,**
**daß** in dem Zahnreinigungsmittel wenigstens ein Tensid aus der Gruppe der Aniontenside enthalten ist.

10. System nach einem der vorangehenden Ansprüche, **dadurch gekennzeichnet,**
**daß** in dem Zahnreinigungsmittel als Feuchthaltemittel wenigstens Sorbitol, Glycerin oder Xylitol bzw. ein beliebiges Gemisch dieser Substanzen enthalten ist.

11. System nach einem der vorangehenden Ansprüche, **dadurch gekennzeichnet,**
**daß** in dem Zahnreinigungsmittel als Poliermittel wenigstens eine Verbindung aus der Gruppe der Kieselsäuren oder Silikate enthalten ist.

12. System nach einem der vorangehenden Ansprüche, **dadurch gekennzeichnet,**
**daß** das Zahnreinigungsmittel zusätzlich wenigstens ein Binde- oder Verdickungsmittel enthält.

13. System nach Anspruch 11, **dadurch gekennzeichnet,**
**daß** das Zahnreinigungsmittel als Binde- oder Verdickungsmittel wenigsten eine wasserunlösliche, nicht-derivatisierte Cellulose enthält.

14. System nach einem der vorangehenden Ansprüche, **dadurch gekennzeichnet,**
**daß** das Zahnreinigungsmittel zusätzlich wenigsten eine Öl/Fett- und/oder Wachskomponente enthält.

15. System nach einem der vorangehenden Ansprüche, **dadurch gekennzeichnet,**
**daß** das Zahnreinigungsmittel als zusätzliche Wirkstoffe Antikarieswirkstoffe, antimikrobielle Wirkstoffe, Zahnstein-Inhibitoren, Remineralisierungswirkstoffe, Geschmacksstoffe oder eine beliebige Kombination dieser Stoffe enthält.

16. System nach einem der vorangehenden Ansprüche, **dadurch gekennzeichnet,**
**daß** das Zahnreinigungsmittel wenigstens
a) 0,3 - 3 Gew.-% eines Tensids oder Tensidgemisches
b) 20 - 85 Gew.-% eines Feuchthaltemittels
c) 5 - 30 Gew.-% eines Poliermittels
d) 0,1 - 5 Gew.-% eines Binde- oder Verdickungsmittels
e) 0,05 - 0,5 Gew.-% eines Fluorids
f) 5 - 50 Gew.-% Wasser und
g) 1 - 6 Gew.-% eines Treibgases oder Treibgasgemisches enthaltend wenigstens eine Komponente, die im Bereich von -15 bis 35 °C siedet,
enthält.

17. Verwendung eines ein treibmittel- und tensidhaltiges Mittel aufnehmenden Spenders mit Mitteln zur Bevorratung und Abgabe des Mittels, wobei die Bevorratungs- und Abgabemittel ausreichend druckstabil gegenüber dem Expansionsdruck des treibmittelhaltigen Mittels bzw. des Treibmittels sind und einen das Zahnreinigungsmittel enthaltenden flexiblen Beutel oder Behälter umfassen, auf den innerhalb des Spenders von außen die Kontraktionskraft eine gedehnten Gummibehälters oder die Expansionskraft eines weiteren, komprimierten Treibgases oder Treibgasgemisches oder die Kraft eines bei oder nach Betätigung des Abgabeventils auf dieses zu bewegten Kolbens einwirken, sowie die Abgabemittel ein eine Abgabeöffnung aufweisendes, manuell zu betätigendes Abgabeventil umfassen, zur Bevorratung und Abgabe eines tensidhaltigen Zahnreinigungsmittels nach Ansprüche 1-16.

18. Verfahren zur Zahnreinigung, **dadurch gekennzeichnet,**
**daß** durch manuelle Betätigung eines Ventils aus einem Spendersystem ein nicht oder nur wenig aufgeschäumter Zahnpastastrang auf einen zur mechanischen Reinigung von Zähnen geeigneten Gegenstand aufgebracht wird, wobei das Zabnreinigungsmittel eine Viskosität größer gleich 30.000 mPas (Brookfield Rotationsviskosimeter RVF, Spindel 3 - 5 bei 4 rpm und 25°C) aufweist und das in dem Zahnreinigungsmittel enthaltene Treibgas oder Treibgasgemisch wenigstens eine Komponente enthält, die bei Normaldruck im Bereich von -15 bis 35° C siedet, so daß der Zahnpastastrang bei 20° C und Normaldruck eine Expansionsrate von nicht mehr als 100 Vol.-% in 5 Sekunden aufweist und dann in einer Zeitspanne von 10 - 100 Sekunden auf nicht mehr als das 6-fache seines Volumens zu einer Zahnpasta-Mousse expandiert, und die gashaltige Zahnpasta-Mousse zur Reinigung von Zahnoberflächen, Zahnzwischenräume und der Mundhöhle verwendet wird.

## Claims

1. A system consisting of a surfactant-containing tooth-cleaning preparation containing
a) at least one polishing component,
b) at least one humectant,
c) at least one surfactant and
d) a propellant, more particularly a propellant gas or mixture of propellant gases,
and a dispenser accommodating the propellant-containing tooth-cleaning preparation and comprising means for storing and dispensing the tooth-cleaning preparation, of which the means for storing and dispensing the tooth-cleaning preparation are designed to be sufficiently pressure-stable towards the expansion pressure of the propellant-containing tooth-cleaning preparation or the propellant and of which means for dispensing the propellant-containing tooth-cleaning preparation comprise a manually operable dispensing valve with a dispensing opening, the tooth-cleaning preparation and the dispenser co-operating in such a way that, when the dispensing valve is manually operated, the propellant-containing tooth-cleaning preparation emerges from the dispensing opening in the form of a lightly foamed strand and foams with delay at the point of application, the tooth-cleaning preparation having a viscosity of, or above, 30,000 mPas (Brookfield RVF rotational viscosimeter, spindle 3-5 at 4 r.p.m./25°C) and the propellant gas or mixture of propellant gases present in the tooth-cleaning preparation containing at least one component which boils at -15 to 35°C at normal pressure.

2. A system as claimed in claim 1, **characterized in that** the storage and dispensing means of the dispenser comprise a flexible container or bag accommodating the tooth-cleaning preparation, on which the contraction force of an expanded rubber container or the expansion force of another compressed propellant gas or mixture of propellant gases or the force of a piston moved towards the dispensing valve during or after operation thereof acts from outside within the dispenser.

3. A system as claimed in claims 1 to 3, **characterized in that** the tooth-cleaning preparation has a low surfactant content of at least 0.3% by weight and at most 3% by weight.

4. A system as claimed in any of the preceding claims, **characterized in that** the tooth-cleaning preparation has a viscosity above 45,000 mPas (Brookfield RVF rotational viscosimeter, spindle 3-5 at 4 r.p.m./25°C).

5. A system as claimed in any of the preceding claims, **characterized in that** the propellant content of the tooth-cleaning preparation is 10% by weight or lower, preferably 4% by weight or lower.

6. A system as claimed in any of the preceding claims, **characterized in that** the tooth-cleaning preparation contains n-butane or isobutane as propellant gas.

7. A system as claimed in any of the preceding claims, **characterized in that** the tooth-cleaning preparation contains a C₅ alkane in a quantity of at least 10% by weight, based on the total quantity of the propellant gas composition, as a propellant gas component.

8. A system as claimed in any of the preceding claims, **characterized in that** the propellant gas mixture present in the tooth-cleaning preparation consists of a mixture of a C₄ alkane and a C₅ alkane.

9. A system as claimed in any of the preceding claims, **characterized in that** at least one surfactant from the group of anionic surfactants is present in the tooth-cleaning preparation.

10. A system as claimed in any of the preceding claims, **characterized in that** at least sorbitol, glycerol or xylitol or a mixture of these substances is present as humectant in the tooth-cleaning preparation.

11. A system as claimed in any of the preceding claims, **characterized in that** at least one compound from the group of silicas or silicates is present as polishing component in the tooth-cleaning preparation.

12. A system as claimed in any of the preceding claims, **characterized in that** the tooth-cleaning preparation additionally contains at least one binder or thickener.

13. A system as claimed in claim 11, **characterized in that** the tooth-cleaning preparation contains at least one water-insoluble, non-derivatized cellulose as binder or thickener.

14. A system as claimed in any of the preceding claims, **characterized in that** the tooth-cleaning preparation additionally contains at least one oil/fat and/or wax component.

15. A system as claimed in any of the preceding claims, **characterized in that** the tooth-cleaning preparation contains anti-caries components, antimicrobial components, scale inhibitors, remineralizing components, flavours or a combination of these substances as additional active components.

16. A system as claimed in any of the preceding claims, **characterized in that** the tooth-cleaning preparation contains at least
a) 0.3 to 3% by weight of a surfactant or surfactant mixture,
b) 20 to 85% by weight of a humectant,
c) 5 to 30% by weight of a polishing component,
d) 0.1 to 5% by weight of a binder or thickener,
e) 0.05 to 0.5% by weight of a fluoride,
f) 5 to 50% by weight of water and
g) 1 to 6% by weight of a propellant gas or mixture of propellant gases containing at least one component which boils at -15 to 35°C.

17. The use of a dispenser accommodating a propellant- and surfactant-containing preparation and comprising means for storing and dispensing the preparation, the storing and dispensing means being designed to be sufficiently pressure-stable towards the expansion pressure of the propellant-containing preparation or the propellant and comprising a flexible container or bag accommodating the tooth-cleaning preparation, on which the contraction force of an expanded rubber container or the expansion force of another compressed propellant gas or mixture of propellant gases or the force of a piston moved towards the dispensing valve during or after operation thereof acts from outside within the dispenser, and the dispensing means comprising a manually operated dispensing valve with a dispensing opening for storing and dispensing a surfactant-containing tooth-cleaning preparation according to claims 1 to 16.

18. A tooth-cleaning process, **characterized in that**, by manual operation of a valve, an unfoamed or only lightly foamed strand of toothpaste is applied from a dispenser system to an article suitable for the mechanical cleaning of teeth, the tooth-cleaning preparation having a viscosity of, or above, 30,000 mPas (Brookfield RVF rotational viscosimeter, spindle 3 - 5 at 4 r.p.m./25°) and the propellant gas or propellant gas mixture present in the tooth-cleaning preparation containing at least one component which boils at -15 to 35°C at normal pressure, so that the toothpaste strand has an expansion rate of no more than 100% by volume in 5 seconds at 20°C/normal pressure and then expands to no more than 6 times its volume in 10 - 100 seconds to form a toothpaste foam and the gas-containing toothpaste foam is used to clean tooth surfaces, interdental spaces and the oral cavity

## Revendications

1. Système constitué par un agent de nettoyage dentaire contenant un ou des agents tensioactifs, contenant
a) au moins un agent de polissage,
b) au moins un agent de rétention de l'humidité,
c) au moins un agent tensioactif et
d) un agent propulseur, en particulier un gaz propulseur ou un mélange de gaz propulseurs,
et un dispositif de distribution contenant l'agent de nettoyage dentaire contenant l'agent propulseur, avec des moyens pour stocker et expulser l'agent de nettoyage dentaire, les moyens pour stocker et expulser l'agent de nettoyage dentaire étant réalisés de manière à être suffisamment résistants à la pression d'expansion de l'agent de nettoyage dentaire contenant un agent propulseur ou de l'agent propulseur, et les moyens pour expulser l'agent de nettoyage dentaire contenant un agent propulseur comprenant une soupape d'expulsion présentant une ouverture d'expulsion, pouvant être actionnée manuellement, l'agent de nettoyage dentaire et le dispositif de distribution interagissant de telle manière que lorsque la soupape d'expulsion est actionnée manuellement, l'agent de nettoyage dentaire contenant un agent propulseur sort de l'ouverture d'expulsion sous forme de brin, peu moussé et mousse de manière retardée dans le temps sur le lieu d'application, l'agent de nettoyage dentaire présentant une viscosité supérieure ou égale à 30000 mPa.s (viscosimètre rotatif de Brookfield RVF, axe 3 - 5 à 4 t/min et 25°C) et le gaz propulseur ou le mélange de gaz propulseurs contenu dans l'agent de nettoyage dentaire contenant au moins un composant qui bout à pression normale dans la plage de -15 à 35°C.

2. Système selon la revendication 1, **caractérisé en ce que** les moyens de stockage et d'expulsion du dispositif de distribution comprennent un récipient ou un sachet souple contenant l'agent de nettoyage dentaire, sur lequel agit, dans le dispositif de distribution, à partir de l'extérieur, la force de contraction d'un récipient en caoutchouc expansé ou la force d'expansion d'un autre gaz propulseur ou mélange de gaz propulseurs comprimé ou la force d'un piston déplacé lors de ou après la manipulation de la soupape d'expulsion vers ledit récipient.

3. Système selon la revendication 1 à 3, **caractérisé en ce que** l'agent de nettoyage dentaire est réalisé avec une basse teneur en agent(s) tensioactif(s), avec une teneur d'au moins 0,3% en poids et d'au maximum 3% en poids.

4. Système selon l'une quelconque des revendications précédentes, **caractérisé en ce que** l'agent de nettoyage dentaire présente une viscosité supérieure à 45000 mPa.s (viscosimètre rotatif de Brookfield RVF, axe 3 à 5 à 4 t/min et 25°C).

5. Système selon l'une quelconque des revendications précédentes, **caractérisé en ce que** la teneur en agent propulseur de l'agent de nettoyage dentaire est inférieure ou égale à 10% en poids, de préférence inférieure ou égale à 4% en poids.

6. Système selon l'une quelconque des revendications précédentes, **caractérisé en ce que** l'agent de nettoyage dentaire contient du n-butane ou de l'isobutane comme gaz propulseur.

7. Système selon l'une quelconque des revendications précédentes, **caractérisé en ce que** l'agent de nettoyage dentaire contient, comme composant du gaz propulseur, un alcane en C₅ en une quantité d'au moins 10% en poids par rapport à la quantité totale de la composition de gaz propulseur.

8. Système selon l'une quelconque des revendications précédentes, **caractérisé en ce que** le mélange de gaz propulseurs contenu dans l'agent de nettoyage dentaire est constitué par un mélange d'un alcane en C₄ et d'un alcane en C₅.

9. Système selon l'une quelconque des revendications précédentes, **caractérisé en ce que** l'agent de nettoyage dentaire contient au moins un agent tensioactif du groupe des agents tensioactifs anioniques.

10. Système selon l'une quelconque des revendications précédentes, **caractérisé en ce que** l'agent de nettoyage dentaire contient, comme agent de rétention de l'humidité, au moins du sorbitol, du glycérol ou du xylitol ou un mélange quelconque de ces substances.

11. Système selon l'une quelconque des revendications précédentes, **caractérisé en ce que** l'agent de nettoyage dentaire contient comme agent de polissage au moins un composé du groupe constitué par les silices ou les silicates.

12. Système selon l'une quelconque des revendications précédentes, **caractérisé en ce que** l'agent de nettoyage dentaire contient en outre au moins un liant ou épaississant.

13. Système selon la revendication 11, **caractérisé en ce que** l'agent de nettoyage dentaire contient comme liant ou épaississant au moins une cellulose insoluble dans l'eau, non transformée en dérivé.

14. Système selon l'une quelconque des revendications précédentes, **caractérisé en ce que** l'agent de nettoyage dentaire contient en outre au moins un composant d'huile/de graisse ou de cire.

15. Système selon l'une quelconque des revendications précédentes, **caractérisé en ce que** l'agent de nettoyage dentaire contient comme substances actives supplémentaires des substances actives anti-caries, des substances actives antimicrobiennes, des inhibiteurs de tartre, des substances actives de reminéralisation, des agents gustatifs ou une combinaison quelconque de ces substances.

16. Système selon l'une quelconque des revendications précédentes, **caractérisé en ce que** l'agent de nettoyage dentaire contient au moins
a) 0,3 - 3% en poids d'un agent tensioactif ou d'un mélange d'agents tensioactifs
b) 20 - 85% en poids d'un agent de rétention de l'humidité
c) 5 - 30% en poids d'un agent de polissage
d) 0,1 - 5% en poids d'un liant ou d'un épaississant
e) 0,05 - 0,5% en poids d'un fluorure
f) 5 - 50% en poids d'eau et
g) 1 - 6% en poids d'un gaz propulseur ou d'un mélange de gaz propulseurs, contenant au moins un composant qui bout dans la plage de -15 à 35 °C.

17. Utilisation d'un dispositif de distribution destiné à recevoir un agent contenant un agent propulseur et un ou des agents tensioactifs, présentant des moyens pour stocker et expulser l'agent, les moyens de stockage et d'expulsion étant suffisamment résistants à la pression d'expansion de l'agent contenant un agent propulseur ou de l'agent propulseur et présentant un sachet ou récipient souple contenant l'agent de nettoyage dentaire, sur lequel agit, dans le dispositif de distribution, à partir de l'extérieur, la force de contraction d'un récipient en caoutchouc expansé ou la force d'expansion d'un autre gaz propulseur ou mélange de gaz propulseurs comprimé ou la force d'un piston déplacé lors de ou après la manipulation de la soupape d'expulsion vers ledit récipient, et les moyens d'expulsion comprenant une soupape d'expulsion, présentant une ouverture d'expulsion, à actionner manuellement, pour stocker et expulser un agent de nettoyage dentaire contenant un ou des agents tensioactifs selon les revendications 1 à 16.

18. Procédé pour le nettoyage des dents, **caractérisé en ce qu'**en actionnant manuellement une soupape, un brin de dentifrice non moussé ou seulement peu moussé est appliqué à partir d'un système de distribution sur un objet approprié pour le nettoyage mécanique des dents, l'agent de nettoyage dentaire présentant une viscosité supérieure ou égale à 30000 mPa.s (viscosimètre rotatif de Brookfield RVF, axe 3 - 5 à 4 t/min et 25°C) et le gaz propulseur ou le mélange de gaz propulseurs contenu dans l'agent de nettoyage dentaire contenant au moins un composant qui bout à pression normale dans la plage de -15 à 35°C de telle manière que le brin de dentifrice présente, à 20°C et à la pression normale un taux d'expansion qui n'est pas supérieur à 100% en volume en 5 secondes et qui ne se développe pas, en un laps de temps de 10 à 100 secondes, à plus de 6 fois son volume en une mousse de dentifrice et la mousse de dentifrice contenant un gaz étant utilisée pour le nettoyage de surfaces dentaires, d'espaces entre les dents et de la cavité buccale.
